# EUROPEAN PATENT APPLICATION

(11) **EP 4 644 525 A1**
(43) Date of publication of application: **05.11.2025**
(21) Application number: 25174126.0
(22) Date of filing: 03.05.2025
(51) Int. Cl.: C12M 1/107, C12M 1/33, C12M 1/00, C02F 1/78, C02F 11/04, C02F 11/06, C12P 5/02, C02F 101/34, C02F 103/32

(54) **BIOGAS PRODUCTION BY ANAEROBIC FERMENTATION**

(30) Priority: 03.05.2024 DE 102024112561
(71) Applicant: VORN Bioenergy GmbH, 93047 Regensburg (DE)
(72) Inventor: Sganzerla, William Gustavo, 93055 Regesburg (DE); Bayerköhler, Jan-Gerd, 93055 Regensburg (DE); Sillero Moreno, Leonor, 93055 Regensburg (DE); Dollinger, Benedikt, 93055 Regensburg (DE)
(74) Representative: Schäfer, Matthias W.

(57) **Abstract**

The present invention relates to a system for biogas production from organic waste materials, comprising an an organic waste material treatment unit; a phenolic compound oxidation unit; an anaerobic fermentation reactor; a digestate solid-liquid separation unit; and a biogas separation and/or utilization unit. Further, a process for biogas production from organic waste materials is disclosed comprising the steps of (a) treating the organic waste materials; (b) oxidizing phenolic compounds in the organic waste materials to form the pre-treated feedstock; (c) subjecting the pre-treated feedstock to anaerobic fermentation to produce biogas; (d) using the biogas directly and/or separating biomethane from any other components of the biogas obtained from the organic waste materials; and (e) separating the solid and liquid components of the digestate. The present invention avoids the drawbacks of the prior art and provides an improved system and process for biogas production by anaerobic fermentation.

## Description

### Field of the Invention

The present invention relates to a system and process for biogas production from organic waste materials.

### Background

Biogas production from organic waste materials is well known in the art. One problem when using organic waste material can be phenolic compounds inhibiting or reducing methanogenesis during fermentation.

The olive crop is one of the most important historical cultures in Mediterranean civilization, and today it is one of the greatest sources of agricultural income in the southern countries of the European Union, North Africa, and the Middle East. After olive oil production, the pulp generated the so called "fresh olive pomace" is submitted to a mechanical extraction process or centrifugation in olive mills. The by-products generated by the centrifugation process are called "olive mill solid waste" and "olive mill wastewater", which the chemical composition is described in the **Table** 1. The composition of the feedstocks may vary depending on the olive's variety, period of collection, annual precipitation, and oil extraction technique applied by the olive mills. However, in general this raw material contains a high amount of phenolic compounds.

**Table 1. Composition of the feedstocks considering in the scope of this patent (fresh olive pomace, olive mill solid waste, and olive mill wastewater).**

| Parameters | Fresh olive pomace | Olive mill solid waste | Olive mill wastewater | Unit |
|---|---|---|---|---|
| Moisture | 72,12 | 84,08 | 90,24 | %FM |
| Stones > 1 mm | 5,97 | 0 | 0 | %FM |
| TS | 27,88 | 15,92 | 9,76 | %FM |
| TVS | 25,79 | 12,82 | 6,31 | %FM |
| Ashes | 2,08 | 3,10 | 3,45 | %FM |
| pH | 5,14 | 4,27 | 4,92 | - |
| tCOD | 185 | 178 | 105 | kg/ton FM |
| sCOD | 120 | 107 | 101 | kg/ton FM |
| Nitrogen | 0,42 | 0,94 | 1,12 | kg/ton FM |
| Phosphorus | 2,3 | 1,52 | 2,07 | kg/ton FM |
| Potassium | 11,64 | 10,97 | 14,99 | kg/ton FM |
| Phenolic compounds | 8,12 | 11,71 | 10,41 | kg/ton FM |
| Phenolic compounds | 0,81 | 1,17 | 1,04 | %/weight FM |

| | | | | |
|---|---|---|---|---|
| Label: FM = fresh matter; TS = total solids; TVS = total volatile solids; sCOD = soluble chemical oxygen demand; tCOD = total chemical oxygen demand. | | | | |

For the application of fresh olive pomace and olive mill by-products in anaerobic digestion process and biogas production, the phenolic compounds can inhibit the methanogenic microorganisms. Most of the phenolic compounds presented in the olive are hydrophilic (soluble in water) and migrate to the olive solid by-products after olive oil extraction. The major phenolic compounds presented in fresh olive pomace and olive mill by-products are classified as phenolic acids (vanillic, cinnamic, p-coumaric, caffeic, gallic, ferulic, and protocatechuic acids), phenolic alcohols (hydroxytyrosol glucoside, hydroxytyrosol, hydroxytyrosol diglucoside, hydroxytyrosol rhamnoside, tyrosol, and tyrosol glucoside), secoiridoids (oleuropein, 10-hydroxy-oleuropein, oleuropein aglycone, oleuropein-aglycone mono-aldehyde, oleacein, oleocanthal and ligstroside aglycon, luteolin-7-glucoside, luteolin, apigenin-7-o-glucoside, apigenin, and rutin), flavonoids (taxifolin, diosmetin, and quercetin), and lignans (hydroxypinoresinol, pinoresinol, and acetoxypinoresinol).

Phenolic compounds are considered refractory organic matter because they inhibit bacterial activity during fermentation, which decreases the methane production, affecting the process efficiency. Therefore, before the application of olive mill by-products in biological process, such as anaerobic digestion, olive mill by-products must undergo a treatment to reduce the concentration of phenolic compounds.

In addition, the reduction of phenolic compounds from fresh olive pomace and olive mill by-products allows for the application of digestate as agricultural fertilizer or animal feed, which is a product generated after biogas production from anaerobic fermentation.

There are known systems and processes to obtain biogas by anaerobic fermentation from olive mills by-products.

EP 1 167 305 A2 describes a process with the application of bentonite. Bentonite is mixed with olive mill wastewater and reduces the chemical oxygen demand over 80% and phenols over 50%. The reduction of chemical oxygen demand during the pretreatment with bentonite is a critical aspect for anaerobic fermentation. The reduction over 80% achieved is not desirable for anaerobic fermentation process, because anaerobic fermentation demands chemical oxygen demand for biomethane production. The phenols are only reduced due to chemical adsorption in the bentonite. EP 1 167 305 A2 mentions that phenols can be submitted to a desorption process to make them available. However, this is not suitable for anaerobic fermentation, since the phenolic compounds could be released during fermentation, inhibiting the microbial activity.

WO 2005/123603 A1 describes a process for treatment of olive mill wastewater. The process applies enzymatic hydrolysis followed by microfiltration, nanofiltration and reverse osmosis. The goal is the production of purified polyphenols that could be applied as nutraceutical and pharmaceutical products. The application of membrane has several disadvantages, such as, cost, maintenance, certain solvents can quickly and permanently destroy the membrane, certain colloidal solids, especially graphite and residues from vibratory deburring operations, can permanently foul the membrane surface.

WO 2009/147693 A2 describes a process for the valorization of digestate generated after fermentation of olive mill wastewater. The digestate is submitted to a process including dilution, enzymatic hydrolysis, microfiltration, and treatment with polymeric resins.

IT TO 2009 0117 A1 describes a process for treatment of olive mill wastewater, applying an integrated system with nanofiltration of the digestate obtained from anaerobic digestion of olive mill by-products for the recovery of digestate. The application of a membrane has several disadvantages, such as, cost, maintenance, certain solvents can quickly and permanently destroy the membrane, certain colloidal solids, especially graphite and residues from vibratory deburring operations, can permanently foul the membrane surface.

### Technical Object

It is therefore the technical object of the present invention to avoid the drawbacks of the prior art and to provide an improved system and process for biogas production by anaerobic fermentation. It is particularly an object of the present invention to provide a system and process for biogas production from organic waste material containing a high amount of phenolic compounds, such as by-products from the olive oil production.

This object has been solved with a system having the features of claim 1 and a process having the features of claim 8.

The present invention provides an integrated system for phenolic compounds removal and biogas production from olive by-products in a sustainable, efficient and cost effective way.

The system for biogas production from organic waste materials according to the present invention comprises:
(a) an organic waste material treatment unit;
(b) a phenolic compound oxidation unit;
(c) an anaerobic fermentation reactor;
(d) a digestate solid-liquid separation unit; and
(e) a biogas separation and/or utilisation unit.

The system according to the present invention could be configured for a multi-stage process, preferably a continuous multi-stage process having the system components arranged preferably in the order as defined by the system claim.

The organic waste material treatment unit is preferably treating the organic waste materials mechanically or by other means breaking the feedstock down, mixing and diluting it with liquid to the target viscosity and or solid content. This unit could be connected to fresh water or recirculated liquid digestate. The organic waste material treatment unit may be working intermittently without affecting the downstream processes.

The organic waste material treatment unit can be connected to the phenolic compound oxidation unit. The phenolic compound oxidation unit is configured to remove phenolic compounds from organic waste material without removing other organic compounds that are important for application in an anaerobic digestion process.

The phenolic compound oxidation unit can be connected to the anaerobic fermentation reactor. In the anaerobic fermentation reactor, the chemical oxygen demand (COD) rich organic waste material coming from the phenolic compound oxidation unit could be exposed to a fermentation process for biogas production.

The anaerobic fermentation reactor can be connected to the solid-liquid separation unit. The liquid digestate could be separated from the solid digestate in the solid-liquid separation unit and recirculated in tubes to a unit at the beginning of the system, e.g. the organic waste material treatment unit, to add liquid if required.

The gaseous sections, mainly containing biomethane and carbon dioxide and being lighter than water, could be separated from the solid and liquid stage in the anaerobic fermentation reactor and flow to a biogas separation unit. The biogas separation unit may be configured to separate the gaseous components containing mainly biomethane and carbon dioxide to obtain biomethane. A utilization unit could be provided for direct use of the obtained biogas.

The present invention provides an advantageous system for reducing the phenolic compounds before fermentation, to avoid methanogenesis inhibition. The organic waste material reacts with oxidizing gas which reduces the phenolic compounds, surprisingly keeping the essential organic nutrients, which are quantified as soluble chemical oxygen demand (sCOD) of the substrate. This means that phenolic compounds are converted in other compounds, such as volatile organic acids, and finally into carbon dioxide and water. Further, the system according to the present invention can advantageously be operated without the application of chemicals, such as bentonite, hydrogen peroxide, iron(II) sulfate, etc., and with the absence of complex and expensive processes such as enzymatic hydrolysis, microfiltration, nanofiltration and reverse osmosis.

An advantageous embodiment of the present invention is characterized in that the organic waste material treatment unit includes equipment for stones separation, crushing, grinding or shredding the waste materials, preferably one or more of olive pomace, olive mill solid waste or olive mill wastewater, to increase their surface area. By this mechanical treatment the surface area of the organic waste may be increased and essential organic nutrients could be efficiently released. The reduced particle size also helps the diffusion during the aeration in the phenolic compound oxidation unit.

Another preferred embodiment is further comprising a buffer tank arranged between the phenolic compound oxidation unit and the anaerobic fermentation reactor. The advantage of including a buffer tank can be to ensure sufficient feedstock to be feed in the subsequent anaerobic fermentation reactor to allow a continuous fermentation process. Also, at this stage additional liquid could be added by recirculated liquid digestate if needed.

Still another advantageous embodiment is characterized in that the phenolic compound oxidation unit comprises a reaction tank and an oxidizing agent unit, the oxidizing agent being preferably an ozone-rich-gas, being most preferably provided by a generator for generating ozone gas. The ozone-rich-gas generator could be connected to the phenolic compound oxidation unit. In the phenolic compound oxidation unit diffusers could be located at the bottom of the tank. The diffusers diffuse the ozone in the tank allowing the organic waste material to react with the oxidation agent. Alternatively, the ozone could be provided by other sources, e.g. an ozone production of a nearby production site or other.

One aspect when pretreating the biomass for anaerobic digestion is the development of processes without loss of essential organic nutrients, which are quantified as soluble chemical oxygen demand (sCOD). Moreover, the addition of chemicals, e.g., iron(II) sulfate and hydrogen peroxide, should be avoided during the pretreatment of substrates for anaerobic digestion since the biological process is susceptible to inhibition with the addition of chemicals. Therefore, for an efficient biogas production from fresh olive pomace and olive mill by-products, the process to remove phenolic compounds could be conducted without the removal of organic matter (sCOD).

A further advantageous embodiment is characterized in that the anaerobic fermentation reactor is configured to maintain a temperature between 20°C and 75°C, preferably a temperature between 35°C and 55°C. This has the special advantage of reduced cooling requirements for systems operating under high temperature ambient conditions.

Another advantageous embodiment is characterized in that the solid-liquid separation unit is configured to separate the solids from the digestate, producing a recirculatable material. In this case, the separation could be obtained e.g. by a screen, a conveyer belt with built in screen, a screw press, a centrifuge, or other suitable devices. The liquid material could then be collected and e.g. be recirculated via pumps, valves and tubes depending on the process needs to the equalization tank or to the buffer tank.

Another advantageous embodiment is characterized in that in the biogas separation and/or utilisation unit methane could be separated from other gases or biogas could be directly provided e.g. to a biogas upgrading unit, a combined heat power unit, a gas turbine producing electric energy, gas burners, gas boilers, gas engines driving an electric generator or supplying mechanical power, fuel cells or any other energy conversion device able to be powered by biogas supplied by the process.

A process for biogas production from organic waste materials according to the present invention comprises the following steps:
(a) treating the organic waste materials;
(b) oxidizing phenolic compounds in the organic waste materials to form the pretreated feedstock;
(c) subjecting the pre-treated feedstock to anaerobic fermentation to produce biogas;
(d) using the biogas directly and/or separating biomethane from any other components of the biogas obtained from the organic waste materials;
   and
(e) separating the solid and liquid components of the digestate.

The process according to the present invention is a multi-stage process, preferably a continuous multi-stage process having the process steps preferably in the order as defined by the process claim.

The treatment of the organic waste is preferably mechanically or by other means breaking the feedstock down, mixing and diluting it with liquid to the target viscosity and or solid content. Also, at this step a) additional liquid could be added by adding fresh water or recirculated liquid digestate.

One aspect of the process for biogas production is that the phenolic compounds are removed from organic waste material for application in an anaerobic digestion process, surprisingly keeping the essential organic nutrients, which are quantified as soluble chemical oxygen demand (sCOD) of the substrate. The process removes the phenolic compounds by oxidation. This means that phenolic compounds are converted in other compounds, such as volatile organic acids, and finally into carbon dioxide and water. As a result, there is not a risk of increasing the phenol concentration during fermentation which is an important achievement and an important advance of anaerobic fermentation process.

After the phenolic compounds removal, the chemical oxygen demand rich feedstock could be applied in a fermentation process with a retention time higher than 20 days, preferably higher than 30 days, which is the suitable time for biogas production. The gaseous sections could be separated from the solid and liquid stage during fermentation. The gaseous components containing mainly biomethane and carbon dioxide being lighter than water will rise to the top of the fermentation tank and could then be separated to obtain biomethane.

The liquid components of the digestate could be separated from the solid components of the digestate and recirculated to a step at the beginning of the process to add liquid if required. The solid digestate could be dried and due to the low phenolic contents could be used for agricultural application.

One advantageous aspect of the inventive process is the absence of chemicals, such as bentonite, hydrogen peroxide, iron(II) sulfate, etc., and with the absence of complex and expensive processes, such as enzymatic hydrolysis, microfiltration, nanofiltration and reverse osmosis in the process.

An advantageous embodiment of the process may include organic waste materials comprising one or more of olive pomace, olive mill solid waste or olive mill wastewater. This advantageously helps to reduce the biological waste from olive oil production. Surprisingly, the olive mill by-products can efficiently be used for biogas production, which the prior art has not considered due to the immanent high share of phenols in the olive mill by-products.

For the application of fresh olive pomace and olive mill by-products in anaerobic digestion process and biomethane production, phenolic compounds, which can inhibit the methanogenic microorganisms are problematic. Most of the phenolic compounds presented in the olive are hydrophilic (soluble in water) and migrate to the olive solid by-products after olive oil extraction. The major phenolic compounds presented in fresh olive pomace and olive mill by-products are classified as phenolic acids (vanillic, cinnamic, p-coumaric, caffeic, gallic, ferulic, and protocatechuic acids), phenolic alcohols (hydroxytyrosol glucoside, hydroxytyrosol, hydroxytyrosol diglucoside, hydroxytyrosol rhamnoside, tyrosol, and tyrosol glucoside), secoiridoids (oleuropein, 10-hydroxy-oleuropein, oleuropein aglycone, oleuropein-aglycone mono-aldehyde, oleacein, oleocanthal and ligstroside aglycon, luteolin-7-glucoside, luteolin, apigenin-7-o-glucoside, apigenin, and rutin), flavonoids (taxifolin, diosmetin, and quercetin), and lignans (hydroxypinoresinol, pinoresinol, and acetoxypinoresinol).

Another advantageous embodiment of the process includes that the organic waste materials are treated in step (a) by grinding or shredding to increase the surface area before oxidizing the phenolic compounds. By increasing the surface area of the organic waste, the essential organic nutrients can be efficiently released.

Yet another advantageous embodiment of the process includes that the oxidizing step (b) is performed by adding an oxidizing agent to the pre-treated feedstock. Preferably the treatment to reduce the concentration of phenolic compounds in the phenolic compound oxidation unit reduces polyphenols to a range of 0,7 % per weight to 0,1 % per weight, preferably 0,5% per weight to 0,3 % per weight in the digestate.

Another advantageous embodiment of the process includes that the oxidizing agent is ozone.

Still another advantageous embodiment of the process includes that the ozone is added to the pre-treated feedstock in a concentration between 0.1%/weight and 10%/weight. This amount of ozone has shown to be effective as an oxidizing agent for removal of phenolic compounds without the addition of other chemical or biological additives.

The process can support the treatment of large volumes of olive mill solid waste and wastewater, the so called olive mill by-products, reducing only the phenolic compounds and keeping the organic fraction high for application in anaerobic digestion. This is because phenolic compounds are considered refractory organic matter inhibiting bacterial activity during fermentation, which decreases the methane production, affecting the process efficiency.

Yet another advantageous embodiment of the process includes that the anaerobic fermentation step (c) is conducted in an anaerobic reactor producing biogas and digestate.

Another advantageous embodiment of the process includes that a solid-liquid separator separates liquid and solid fractions of the digestate.

The digestate obtained after anaerobic digestion is separated by a solid-liquid separator, such as for example a screw press, and the liquid fraction may be recirculated in the process.

Still another advantageous embodiment of the process includes that the liquid fraction could be recirculated to the equalization tank in step (a) to solubilize the fresh olive pomace, olive mill solid waste and/or olive mill wastewater.

A further advantageous embodiment of the process includes that liquid digestate could be recirculated to a buffer tank arranged between the phenolic compound oxidation unit and the anaerobic fermentation reactor.

Yet a further advantageous embodiment of the process includes that the phenolic compound oxidation unit comprises a reaction tank and an ozone-rich-gas generator which generates ozone gas. Alternatively, the ozone could be provided by other sources, e.g. ozone production of a nearby production site.

A product resulting from the inventive method is a mixture of methane and other gases, and has a higher heating value than natural gas. The gaseous components containing mainly biomethane and carbon dioxide could be separated to obtain biomethane.

Further the present invention provides for the use of the digestate obtained in the inventive process as an agricultural fertilizer.

The reduction of phenolic compounds from fresh olive pomace and olive mill by-products allows for the application of digestate as agricultural fertilizer, which could be products generated after biomethane production from anaerobic fermentation. This could again improve the efficiency of the inventive process.

### Brief Description of the Drawings

The present invention will become more apparent in connection with the attached drawings.
- **Figure 1**: Block diagram of a system for biogas production from organic waste materials according to the present invention;
- **Figure 2**: Flowchart of a process for biogas production from organic waste materials according to the present invention;
- **Figure 3**: Flowchart of a preferred embodiment of the process according to figure 1.
- **Figure 4**: Experimental results regarding the application of the feedstock "fresh olive pomace" in the advance oxidation process for the specific removal of phenolic compounds. The results are expressed based on fresh matter of olive pomace;
- **Figure 5**: Experimental results regarding the application of the feedstock "fresh olive pomace" in the advance oxidation process for the specific removal of phenolic compounds. The results are expressed based on organic dry matter of olive pomace;
- **Figure 6**: Experimental results regarding the profile of ozone rich-gas applied for the treatment of the feedstock "fresh olive pomace" in the advance oxidation process for the specific removal of phenolic compounds.
- **Figure 7**: Experimental results regarding the anaerobic fermentation of "fresh olive pomace", tested with and without the application of the proposed advanced oxidation process for the specific removal of phenolic compounds.
- **Figure 8**: Experimental results regarding the application of the feedstock "olive mill solid waste" in the advance oxidation process for the specific removal of phenolic compounds. The results are expressed based on fresh matter of olive pomace;
- **Figure 9**: Experimental results regarding the application of the feedstock "olive mill solid waste" in the advance oxidation process for the specific removal of phenolic compounds. The results are expressed based on organic dry matter of olive pomace;
- **Figure 10**: Experimental results regarding the profile of ozone rich-gas applied for the treatment of the feedstock "olive mill solid waste" in the advance oxidation process for the specific removal of phenolic compounds.
- **Figure 11**: Experimental results regarding the anaerobic fermentation of "olive mill solid waste", tested with and without the application of the proposed advanced oxidation process for the specific removal of phenolic compounds.
- **Figure 12**: Experimental results regarding the application of the feedstock "olive mill wastewater" in the advance oxidation process for the specific removal of phenolic compounds. The results are expressed based on fresh matter of olive pomace;
- **Figure 13**: Experimental results regarding the application of the feedstock "olive mill wastewater" in the advance oxidation process for the specific removal of phenolic compounds. The results are expressed based on organic dry matter of olive pomace;
- **Figure 14**: Experimental results regarding the profile of ozone rich-gas applied for the treatment of the feedstock "olive mill wastewater" in the advance oxidation process for the specific removal of phenolic compounds.
- **Figure 15**: Experimental results regarding the anaerobic fermentation of "olive mill wastewater", tested with and without the application of the proposed advanced oxidation process for the specific removal of phenolic compounds.

### Detailed Description of the Drawings

Following a process for biogas production is described which comprises the steps of equalization or mechanical treatment of the organic waste material, providing ozone, injection of ozone in the organic waste material, equalization, anaerobic fermentation for biogas production, solid-liquid separation of the digestate and optional recirculation of the liquid digestate in the pretreatment process. One advantageous aspect is associated with the specific removal of phenolic compounds from fresh olive pomace and olive mill by-products (solid waste and wastewater), which allow the application for biogas production. Surprisingly, the application of this process allows the increase in the biogas production from fresh olive pomace and olive mill by-products, maximizing the resources utilization for renewable energy production.

The process for biogas production integrates ozone-based removal of phenolic compounds from organic waste materials, e.g. fresh olive pomace and olive mill by-products (solid waste and wastewater), with the possibility to treat recirculation of effluent generated after the anaerobic digestion (liquid digestate), creating a recirculation system that maximizes the ozone utilization and phenolic compounds removal. The process only targets phenolic compounds, without removing other organic compounds that are important for anaerobic fermentation (expressed as sCOD). The principle of the advanced oxidation process of the present invention is the generation of highly oxidizing radicals, especially the hydroxyl radical capable of oxidizing high recalcitrant contaminants (phenolic compounds) to carbon dioxide and water. When phenolic compounds are chemically oxidized, the cyclic ring is split into smaller molecules such as dicarboxylic acids (oxalic acid, glyoxalic acid, glyoxal, and formic acid) before ultimately converted to carbon dioxide with continued oxidation. This means that the proposed process enhanced the biodegradability of the fresh olive pomace and olive mill by-products, allowing a more efficient utilization of the biomass for the microbial processes, promoting a sustainable approach to pollutant removal and biogas production.

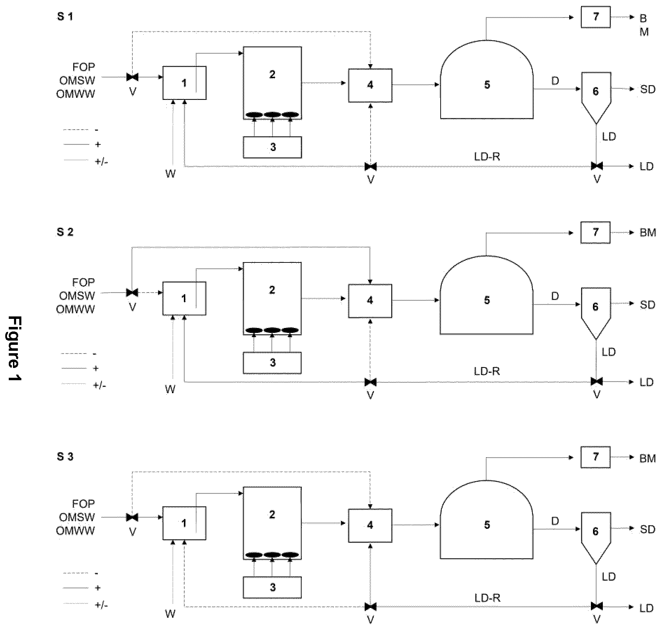

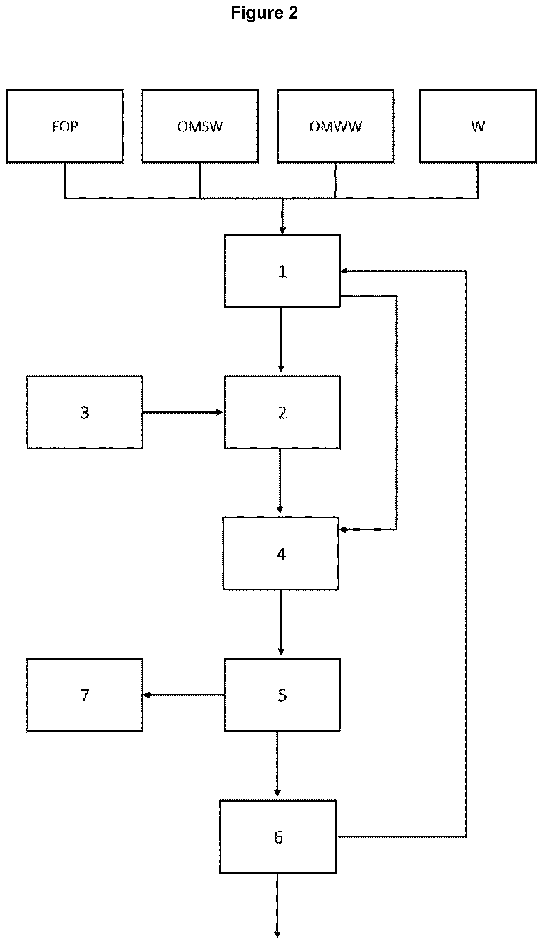

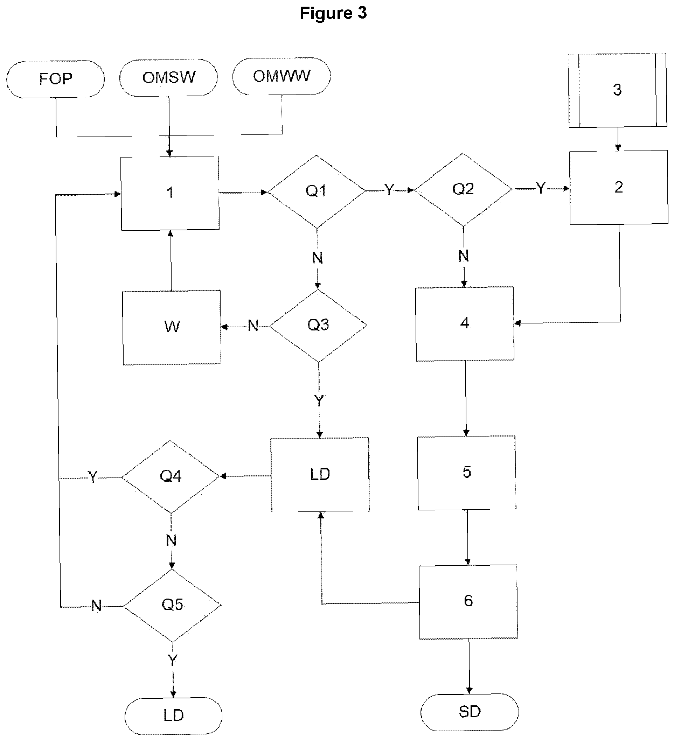

In the following steps, a detailed process overview was provided, where each topic refers to the system block diagram of Figure 1 and the related process flowchart presented in Figure 2. In addition, Figure 3 presents a flowchart for the operation of the process, considering the main steps associated with general flowchart.

**Step 1)** The process begins with the mixture of feedstock (fresh olive pomace and olive mill by-products) in an equalization tank 1. The feedstock can be treated individually or in a mixture. All the proportions between fresh olive pomace and olive mill by-products may be allowed in the process, this means that the feedstocks could be treated as a mixture flow or as individual flow.

The pH value of the mixture could be neutralized by adding non-reactive substances, e.g., lime, to reach a pH value between 6.5 and 7.5. The equalization tank 1 can be covered to avoid emissions of any gas formed in this step. The equalization tank 1 may be equipped with mixers to ensure homogenization of the raw material and to prevent the deposition of solid material on the bottom of the equalization tank 1.

The feedstock may be mixed with water or liquid digestate obtained from the separation of the digestate as will be described later in step 6, until a preferable viscosity for aeration is achieved. The preferable viscosity is automatically determined by the process based on the pressure adopted for ozonation in the reaction tank 2 with ozone provided from an ozone-rich gas generator 3. This process step will be described in further detail in step 2. However, if the concentration of phenolic compounds is below a required threshold value for the process which is adjustable for each application, the feedstock can be sent directly to the buffer tank 4. This will be described in further detail in step 4.

**Step 2)** The mixture obtained from feedstock and water or liquid digestate may be submitted to advanced oxidation by aerating with ozone-rich gas having an average input concentration of 90 g O₃/Nm³ O₂. This process aims to reduce the phenolic compounds and possibly also odors present in the olive mill solid waste and wastewater.

The process also aims to increase the availability of nutrients for fermentation. Ozone, a powerful oxidizing agent, may be introduced in the mixture obtained from feedstock and water or liquid digestate as described in step 1 to facilitate the breakdown of organic contaminants and pathogens. The oxidation step may improve the biodegradability of fresh olive pomace and olive mill by-products, allowing for subsequent treatment stages being more effective for fermentation and biogas production.

The organic waste material, coming from the equalization tank 1 (step 1), is pumped e.g. by a screw conveyer, sludge pump or similar through the upper part of the reaction tank 2 countercurrent with the ozone-rich gas and may be collected at the bottom of the reaction tank 2.

After ozone production in the ozone-rich-gas generator 3 (step 3), the ozone gas is diffused by preferably titanium diffusers located at the bottom of the reaction tank 2 and connected with the ozone-rich-gas generator 3. The reaction tank 2 may be covered to avoid air emissions.

At the top of the reaction tank 2, a thermocatalytic ozone destructor (not shown) may be installed on top of the reaction 2 to prevent the release of ozone gas or odor to the atmosphere. The end-product obtained from the ozone destructor is oxygen. The gas-liquid interaction of ozone-rich gas with the fresh olive pomace and olive mill by-products will oxidize the phenolic compounds contained in the fresh olive pomace and olive mill by-products.

The following operating conditions should preferably be applied for the efficient removal of specific phenolic compounds: ozone dosage between 1 kg O₃/ton and 6 kg O₃/ton feedstock, preferably 1,8 kg O₃/ton fresh olive pomace, 2,7 kg O₃/ton olive mill solid waste, and 5,5 kg O₃/ton olive mill wastewater; temperature between 20 °C and 55 °C, preferably 35 °C; pH value between 6 and 9, preferably 7,5; and retention time in step 2 between 1 h and 3 h, preferably 2 h.

The operating conditions, i.e. type of feedstock, ozone dosage, temperature, pH, and time, could be automatically monitored by respective sensors and indicated to a control system and may be adjusted by a programmable logic controller having respective settings stored in the memory.

Therefore, the removal efficiency of phenolic compounds (% removed) is monitored by the system and depending on the target reduction or threshold value established for the process, the operating conditions are adjusted to promote a cost- and energy-efficient reduction of phenolic compounds.

**Step 3)** Ozone-rich gas is produced *in situ* by an ozone generator considering a continuous operation during 24 h/day. Ozone-rich gas is produced from oxygen in a ozone-rich-gas generator 3 that is commercially available. The ozone-rich-gas generator 3 may automatically be monitored and adjusted with a programmable logic controller. Therefore, depending on the characteristics of the initial feedstock, different dosage of ozone may be applied in the process.

The oxygen required for the ozone-rich-gas generator 3 may be produced *on site* from ambient air by an oxygen station installed with the ozone-rich-gas generator 3. Alternatively, liquid oxygen having 95-99% purity may be used to feed the ozone-rich-gas generator 3. Alternatively, the oxygen could be recycled after the thermocatalytic ozone destructor, where the end-product obtained from the ozone destructor is oxygen. For this, the purification with activated carbon might be necessary to remove impurities obtained from the aeration.

The ozone-rich-gas generator 3 may be based on a ferrule-type generation core with borosilicate dielectric, and may be mainly made of stainless steel.

**Step 4)** After applying ozone to the fresh olive pomace and olive mill by-products in the reaction tank 2, the pre-treated material with reduced content of phenolic compounds, coming from the reaction tank 2 may be accumulated in a buffer tank 4. The buffer tank 4 has the purpose to ensure that the continuous process has sufficient pre-treated feedstock to feed the following anaerobic reactor 5. In the buffer tank 4, raw feedstock may be added to ensure suitable conditions for fermentation. The buffer time could be one or several days or more.

The buffer tank 4 may be equipped with mixers to prevent the deposition of solid material on the bottom of the tank.

**Step 5)** The pre-treated material with reduced content of phenolic compounds is submitted to the fermenter for anaerobic fermentation for biomethane production. In this step, the pre-treated material or organic matter undergoes microbial breakdown in absence of oxygen, producing biogas and digestate (effluent).

Since the phenolic compound concentration was previously lowered by oxidation in the ozone treatment step, the microbial inhibition which can be caused by elevated phenolic compounds is lowered, thus enhancing methanogenic activity. In addition, the reduction of phenolic compounds enhances the methane yield from methanogenic anaerobic fermentation of fresh olive pomace and olive mill by-products. This means that more biomethane can be produced from the same amount of feedstock, increasing the efficiency of anaerobic fermentation.

**Step 6)** The digestate obtained by the process is pumped from the digesters to the solid-liquid separator 6. In the solid-liquid separator 6 the digestate may be pressed through a screen and liquid and solid fractions are separated. At the end of process step 6, the liquid fraction may be recirculated to the equalization tank 1 to solubilize the fresh olive pomace and/or olive mill by-products.

Liquid digestate separated in the solid-liquid separator 6 may alternatively be recirculated to the buffer tank 4. This may be useful if the phenolic compounds are below the threshold value already at the beginning and specifications for the fermentation process allow the raw material directly to be sent to the buffer tank 4.

The recirculation of the liquid digestate enhances the overall efficiency of the treatment process by decreasing the demand of fresh water. However, fresh water may be used in the process, if available, and depending on the parameters of the operating conditions for the pretreatment and fermentation.

In the case where liquid digestate is not available for recirculation, fresh water may be necessary to dilute the fresh olive pomace and/or olive mill by-products. In addition, if the concentration of phenolic compounds is higher than a threshold value, the digestate can be recirculated in the process for the removal of phenolic compounds. This way, liquid digestate may be used as fertilizer if the threshold values of phenolic compounds for the application as agricultural fertilizer are met.

### Example

The process according to the present invention was tested in a continuous system.

Three separate tests were conducted with I) olive pomace, II) olive mill solid waste, and III) olive wastewater respectively.

The raw material was mechanically treated by stone separation, crushing, and shredding the waste materials thereby increasing their surface area.

Then ozone was in situ produced and injected with aerator diffusers in a cylindrical tank. The diffusers were evenly distributed at the bottom of the tank and connected with an ozone generation unit. The process has an average retention time of 150 min, which means that the time to complete the oxidation of fresh olive pomace and olive mill by-products inside the contact tank is 150 min. In the figures, the process was tested during two retentions time (300 min). The results obtained indicated that the input concentration of phenolic compounds was always standard during the oxidation.

After the continuous oxidation, the concentration of phenolic compounds decreased, reaching a constant percentage of reduction. In addition, the sCOD of the end-product (pretreated feedstock) presented a stable concentration, without removal during the process. Moreover, the profile of ozone (input, output, and used) applied during the treatment of fresh olive pomace and olive mill by-products was constant, where most of the ozone injected in the tank is used for the removal of phenolic compounds.

Finally, the feedstock pretreated with the present invention was submitted to biochemical methane tests according to VDI 4630, and the same test was performed with the feedstocks without the proposed invention (control sample).

The results indicated that the application of the present invention increases the biomethane production in all three cases:
I) Fresh olive pomace: In the case of fresh olive pomace the methane yield increased 20,39%: from 255 Nm³ CH₄/kg TVS (control) to 307 Nm³ CH₄/kg TVS (with the present invention).
II) Olive mill solid waste: In the case of olive mill solid waste the methane yield increased 21%: from 280 Nm³ CH₄/kg TVS (control) to 340 Nm³ CH₄/kg TVS (with the present invention).
III) Olive mill wastewater: In the case of olive mill wastewater the methane yield increased 45,41%: from 447 Nm³ CH₄/kg TVS (control) to 650 Nm³ CH₄/kg TVS (with the present invention).

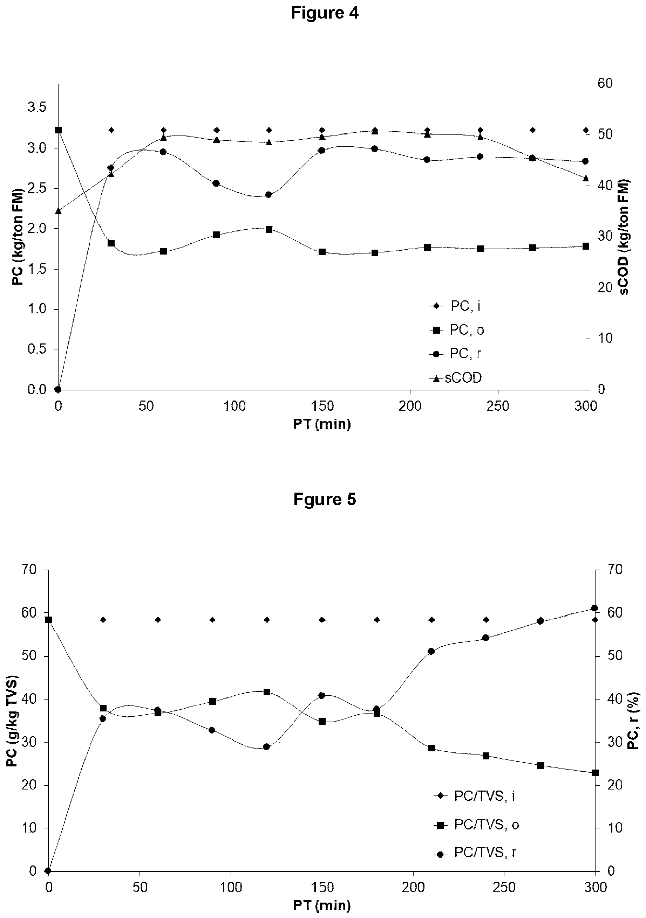

Figure 4 shows the experimental results for the removal of phenolic compounds from fresh olive pomace (FOP). The figure shows a constant feeding of FOP, with a stable concentration of phenolic compounds in the input material (PC, i). The main achievement obtained is associated with the injection of ozone rich gas under the conditions mentioned above in the patent, and the reduction of phenolic compounds in the output material (PC, o). We can clearly see that the output material has a stable profile, meaning that the developed process can be suitable for full-scale implementation. The phenolic compounds reduced (PC, r) was expressed as % of fresh matter of input material, and the results obtained for FOP demonstrated a reduction around 45% of phenolic compounds. Higher reductions can be obtained adjusting the dose of ozone in the process; however, this will lead to higher operational costs (electricity). Finally, the stability of organic fraction (sCOD) was investigated over the reaction time. The results demonstrated an initial increase of the sCOD (from 0 to 100 min), which is associated with the degradation of total organic compounds into soluble. After this initial degradation, there is a stabilization of the sCOD, followed by a slight reduction in the end. However, comparing the initial with the final sCOD, no reduction was observed, demonstrating that the process is feasible for future biogas production.

Figure 5 shows the experimental results for the removal of phenolic compounds from fresh olive pomace (FOP), however, the results are being expressed based on the amount of TVS (total volatile solids). This approach was used to verify the ratio between phenolic compounds and TVS, which is a parameter widely used for the modelling of biogas process. PC/TVS ratio around or below 30 g/kg are recommended for biogas production (mono-fermentation). The FOP presented an initial PC/TVS ratio of 60 g/kg, which is not suitable for biogas production, however, after the application of the new process, the ratio was reduced to around 25 g/kg. This represents that FOP after the application of the new process, can be used as a mono-fermentation substrate when considering only the limits of phenols as parameters. Finally, the results obtained corroborate Figure 4, where the phenolic compounds were reduced over the pretreatment time. In this case, the reduction of phenolic compounds based on TVS reached 60%, demonstrating again, a feasible approach for pretreatment and fermentation.

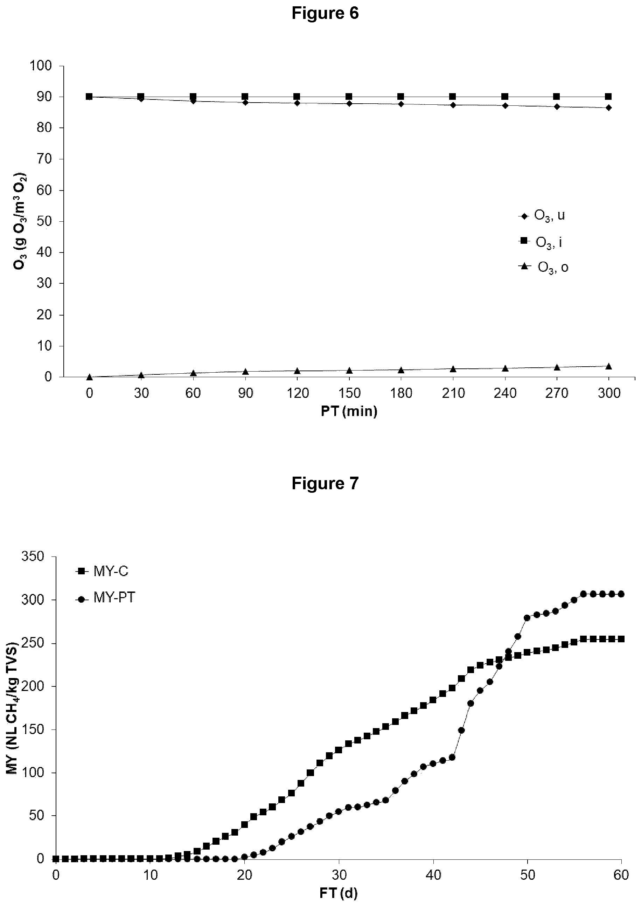

Figure 6 shows the profile of ozone in the process. Ozone rich gas is injected in the bottom of the reaction tank, and the goal is that ozone reacts with the phenolic compounds and converts into other organic substrates (organic acids, carbon dioxide, water). Therefore, a stable ozone profile is necessary to achieve an efficient and profitable process. In this study, the ozone injected was consumed in the reaction tank, and only a small fraction of ozone was released from the reaction tank. The released ozone is treated in an ozone destroyer unit, to avoid air emissions. Therefore, the profile of ozone injected, used and released demonstrates that most of the ozone was used for the pretreatment of phenolic compounds, with an efficient utilization during the process.

Figure 7 shows the biochemical methane potential of the FOP. In this study, the methane production was compared between the FOP without pretreatment (control) and FOP after the proposed pretreatment. The results obtained indicate that the application of the new process can increase the biogas production up to 20,39%: from 255 Nm³ CH₄/kg TVS (control) to 307 Nm³ CH₄/kg TVS (with the present invention). The application of the new pretreatment can remove/oxidize the phenolic compounds, converting the substrate FOP into a more biodegradable for the methanogenic microorganisms, increasing the efficiency ad yield of biogas production.

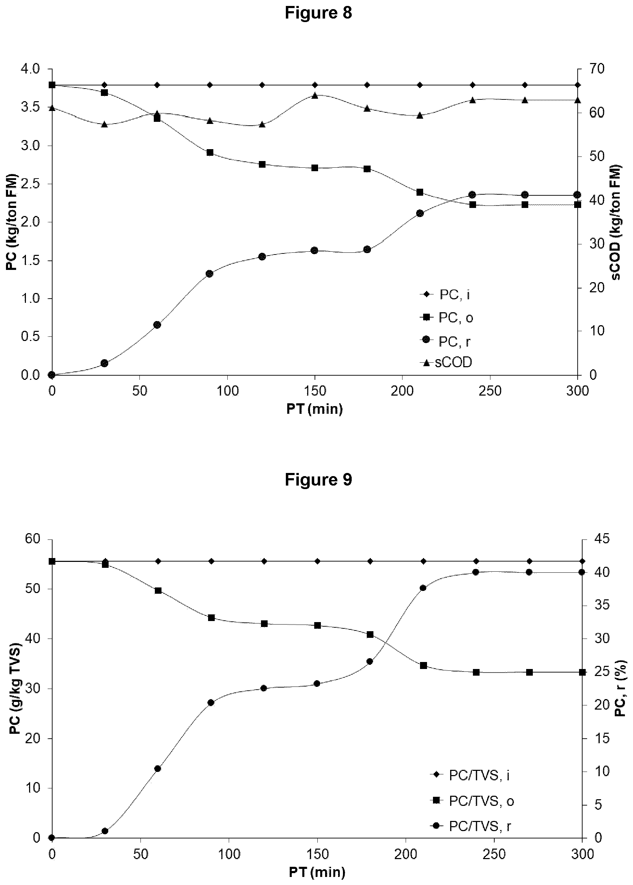

Figure 8 shows the experimental results for the removal of phenolic compounds from olive mill solid waste (OMSW). The figure shows a constant feeding of OMSW, with a stable concentration of phenolic compounds in the input material (PC, i). The main achievement obtained is associated with the injection of ozone rich gas under the conditions mentioned above in the patent, and the reduction of phenolic compounds in the output material (PC, o). The phenolic compounds reduced (PC, r) was expressed as % of fresh matter of input material, and the results obtained for OMSW demonstrated a reduction around 40% of phenolic compounds. Higher reductions can be obtained adjusting the dose of ozone in the process; however, this will lead to higher operational costs (electricity). Finally, the stability of organic fraction (sCOD) was investigated over the reaction time. The results demonstrated a stabilization of the sCOD. Comparing the initial with the final sCOD, no reduction was observed, demonstrating that the process is feasible for future biogas production.

Figure 9 shows the experimental results for the removal of phenolic compounds from OMSW, however, the results are being expressed based on the amount of TVS (total volatile solids). This approach was used to verify the ratio between phenolic compounds and TVS, which is a parameter widely used for the modelling of biogas process. PC/TVS ratio around or below 30 g/kg are recommended for biogas production (mono-fermentation). The OMSW presented an initial PC/TVS ratio of 55 g/kg, which is not suitable for biogas production, however, after the application of the new process, the ratio was reduced to 32 g/kg. This represents that OMSW after the application of the new process, can be used as a mono-fermentation substrate when considering only the limits of phenols as parameters. Finally, the results obtained corroborate Figure 8, where the phenolic compounds were reduced over the pretreatment time. In this case, the reduction of phenolic compounds based on TVS reached 40%, demonstrating again, a feasible approach for pretreatment and fermentation.

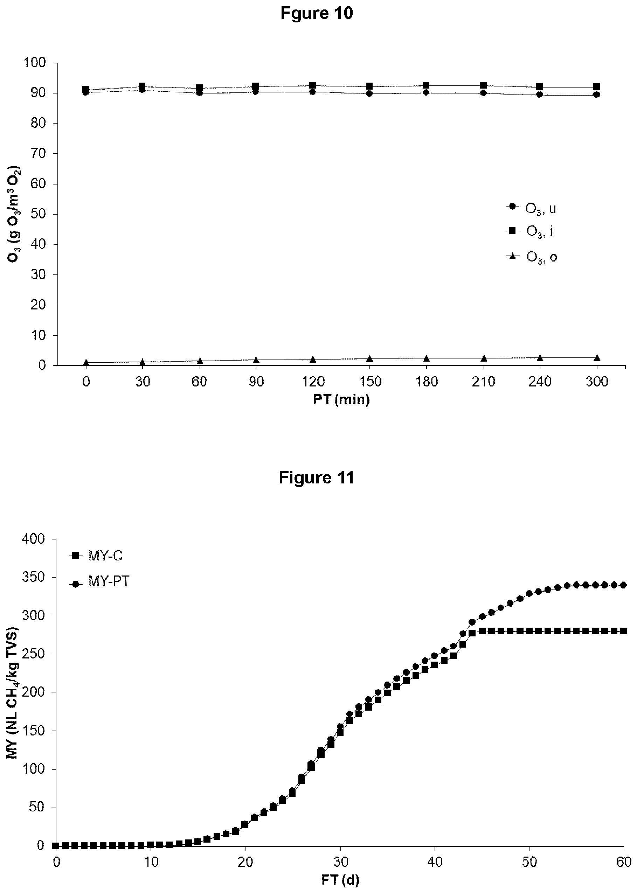

Figure 10 shows the profile of ozone in the process. Ozone rich gas is injected in the bottom of the reaction tank, and the goal is that ozone reacts with the phenolic compounds and converts into other organic substrates (organic acids, carbon dioxide, water). Therefore, a stable ozone profile is necessary to achieve an efficient and profitable process. In this study, the ozone injected was consumed in the reaction tank, and only a small fraction of ozone was released from the reaction tank. The released ozone is treated in an ozone destroyer unit, to avoid air emissions. Therefore, the profile of ozone injected, used and released demonstrates that most of the ozone was used for the pretreatment of phenolic compounds, with an efficient utilization during the process.

Figure 11 shows the biochemical methane potential of the OMSW. In this study, the methane production was compared between the OMSW without pretreatment (control) and OMSW after the proposed pretreatment. The results obtained indicate that the application of the new process can increase the biogas production up to 21,4%: from 280 Nm³ CH₄/kg TVS (control) to 340 Nm³ CH4/kg TVS (with the present invention). The application of the new pretreatment can remove/oxidize the phenolic compounds, converting the substrate OMSW into a more biodegradable for the methanogenic microorganisms, increasing the efficiency ad yield of biogas production.

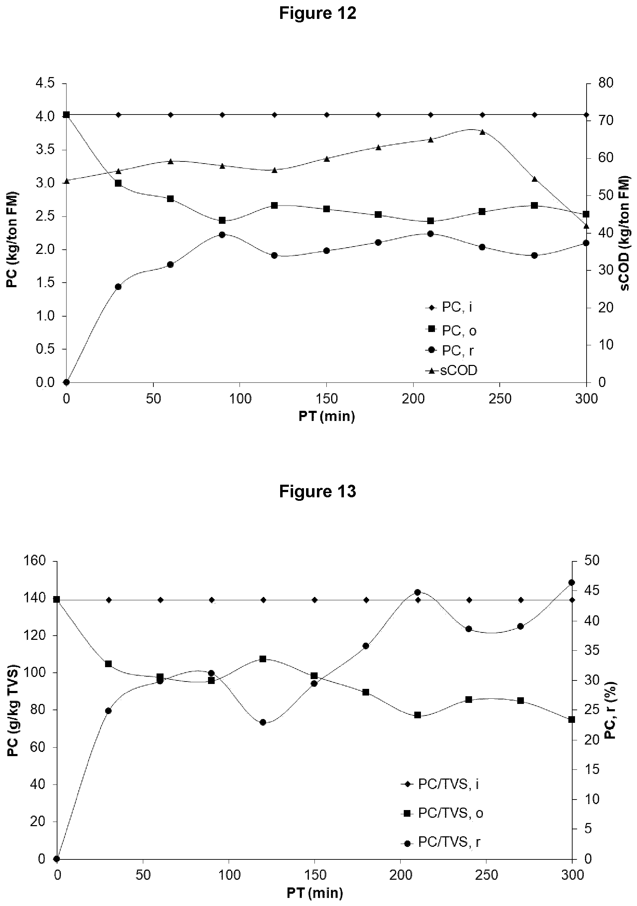

Figure 12 shows the experimental results for the removal of phenolic compounds from olive mill wastewater (OMWW). The figure shows a constant feeding of OMWW, with a stable concentration of phenolic compounds in the input material (PC, i). The main achievement obtained is associated with the injection of ozone rich gas under the conditions mentioned above in the patent, and the reduction of phenolic compounds in the output material (PC, o). We can clearly see that the output material has a stable profile, meaning that the developed process can be suitable for full-scale implementation. The phenolic compounds reduced (PC, r) was expressed as % of fresh matter of input material, and the results obtained for OMWW demonstrated a reduction around 38% of phenolic compounds. Higher reductions can be obtained adjusting the dose of ozone in the process; however, this will lead to higher operational costs (electricity). Finally, the stability of organic fraction (sCOD) was investigated over the reaction time. Comparing the initial with the final sCOD, a small reduction was observed, without negative effect on the feasible for future biogas production.

Figure 13 shows the experimental results for the removal of phenolic compounds from OMWW, and the results are being expressed based on the amount of TVS (total volatile solids). This approach was used to verify the ratio between phenolic compounds and TVS, which is a parameter widely used for the modelling of biogas process. PC/TVS ratio around or below 30 g/kg are recommended for biogas production (mono-fermentation). The OMWW presented an initial PC/TVS ratio of 140 g/kg, which is not suitable for biogas production, however, after the application of the new process, the ratio was reduced to below 70 g/kg. This represents an improvement of the quality of the OMWW for biogas, and in this case, the substrate after the application of the new process can be used as a co-fermentation substrate with the other olive mill by-products. However, additional ozone dose can be applied to reduce the phenolic compounds of OMWW to the limits below 30 g/kg, for application as mono-fermentation substrate when considering only the limits of phenols as parameters. Finally, the results obtained corroborate Figure 12, where the phenolic compounds were reduced over the pretreatment time. In this case, the reduction of phenolic compounds based on TVS reached around 45%, demonstrating again, a feasible approach for pretreatment and fermentation.

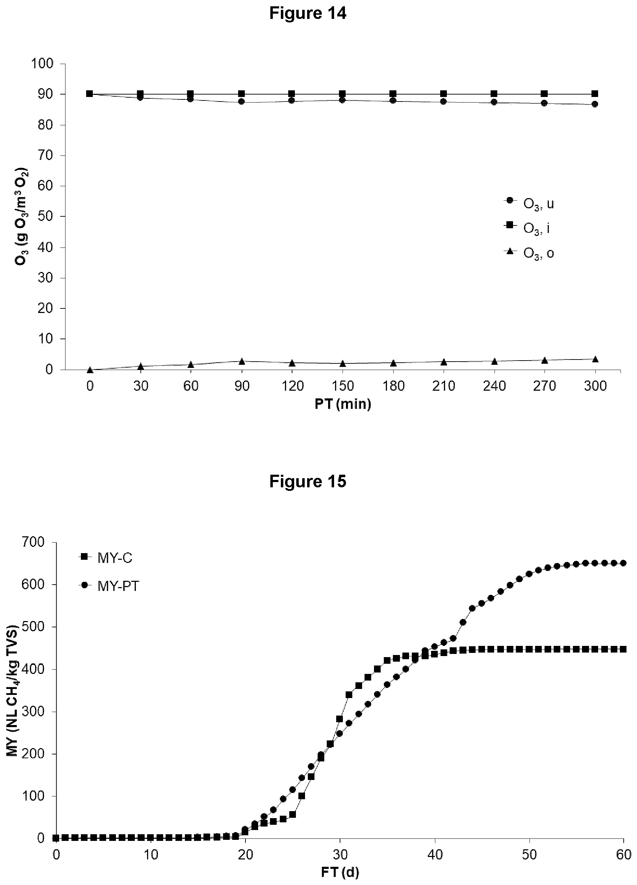

Figure 14 shows the profile of ozone in the process. Ozone rich gas is injected in the bottom of the reaction tank, and the goal is that ozone reacts with the phenolic compounds and converts into other organic substrates (organic acids, carbon dioxide, water). Therefore, a stable ozone profile is necessary to achieve an efficient and profitable process. In this study, the ozone injected was consumed in the reaction tank, and only a small fraction of ozone was released from the reaction tank. The released ozone is treated in an ozone destroyer unit, to avoid air emissions. Therefore, the profile of ozone injected, used and released demonstrates that most of the ozone was used for the pretreatment of phenolic compounds, with an efficient utilization during the process.

Figure 15 shows the biochemical methane potential of the OMWW. In this study, the methane production was compared between the OMWW without pretreatment (control) and OMWW after the proposed pretreatment. The results obtained indicate that the application of the inventive process can increase the biogas production up to 45%: from 447 Nm³ CH₄/kg TVS (control) to 650 Nm³ CH₄/kg TVS (with the present invention). The application of the new pretreatment can remove/oxidize the phenolic compounds, converting the substrate OMWW into a more biodegradable for the methanogenic microorganisms, increasing the efficiency ad yield of biogas production.

The invention is not limited to the preferred embodiments or examples given above. Rather, a number of variants are conceivable, which also make use of the solution described in fundamentally different embodiments. For example a mixture of FOP, OMSW and OMWW could be used as well.

### Reference Numerals

1 Equalization tank;
2 Reaction tank;
3 Ozone-rich-gas generator;
4 Buffer tank;
5 Fermenter;
6 solid/liquid separator.
PC, phenolic compounds
FM, fresh matter
i, input
o, output
r, reduction
u, used
sCOD, soluble chemical oxygen demand
TVS, total volatile solids
t, time
PC/TVS, phenolic compounds/total volatile solids
O₃, ozone
MY, methane yield
MY-C, methane yield control (without pretreatment)
MY-PT, methane yield with pretreatment
FT, fermentation time
PT, pretreatment time
D, digestate
SD, solid digestate
LD, liquid digestate
LD-R, liquid digestate recirculate
BM, biomethane
S, scenario
V, valve
d, days
kg, kilogram
ton, tonnes
min, minutes
FOP, fresh olive pomace
OMSW, olive mill solid waste
OMWW, olive mill wastewater
Y, yes
N, no
+, on
-, off
+/-, optional
Q1, Is the viscosity suitable for aeration?
Q2, Is the phenols concentration suitable for the process?
Q3, Is recirculate available?
Q4, Is recirculate required in the process?
Q5, Is the phenols concentration suitable for fertilizer application?

## Claims

1. A system for biogas production from organic waste materials, comprising:
(a) an organic waste material treatment unit;
(b) a phenolic compound oxidation unit;
(c) an anaerobic fermentation reactor;
(d) a digestate solid-liquid separation unit; and
(e) a biogas separation and/or utilization unit.

2. The system of claim 1, wherein the organic waste material treatment unit includes equipment for stones separation, crushing, grinding or shredding the waste materials, preferably one or more of olive pomace, olive mill solid waste or olive mill wastewater to increase their surface area.

3. The system of claim 1 or 2, further comprising a buffer tank arranged between the phenolic compound oxidation unit and the anaerobic fermentation reactor.

4. The system of claim 1, 2 or 3, wherein the phenolic compound oxidation unit comprises a reaction tank and an oxidizing agent unit, the oxidizing agent being preferably an ozone-rich-gas, being most preferably provided by a generator for generating ozone gas.

5. The system of any of claim 1 - 4, wherein the anaerobic fermentation reactor is configured to maintain a temperature between 20°C and 75°C.

6. The system of any of claim 1 - 5, wherein the solid-liquid separation unit is configured to separate the solids from the digestate, producing a recirculatable material.

7. The system of any of claim 1 - 6, wherein the biogas separation unit is configured to separate the methane from other gases.

8. A process for biogas production from organic waste materials, comprising the following steps:
(a) treating the organic waste materials;
(b) oxidizing phenolic compounds in the organic waste materials to form the pretreated feedstock;
(c) subjecting the pre-treated feedstock to anaerobic fermentation to produce biogas;
(d) using the biogas directly and/or separating biomethane from any other components of the biogas obtained from the organic waste materials;
and
(e) separating the solid and liquid components of the digestate.

9. The process of claim 8, wherein the organic waste materials comprise one or more or a mixture of olive pomace, olive mill solid waste or olive mill wastewater.

10. The process of any of claim 8 or 9, wherein the organic waste materials are treated in step (a) by grinding or shredding to increase the surface area before oxidizing the phenolic compounds.

11. The process of any of claim 8 to 10, wherein the oxidizing step (b) is performed by adding an oxidizing agent, preferably ozone, to the pre-treated feedstock.

12. The process of claim 11, wherein the ozone is added to the pre-treated feedstock in an amount of 0.1%/weight to 10%/weight.

13. The process of any of claim 8 to 12, wherein the liquid fraction obtained in the solid-liquid separator is recirculated to step (a) to solubilize the organic waste material or to a buffer tank arranged between the phenolic compound oxidation unit and the anaerobic fermentation reactor.

14. Use of the digestate obtained in the process of any of claim 8 to 13 as an agricultural fertilizer.
